# EUROPÄISCHE PATENTSCHRIFT

(11) **EP 2 621 617 B1**
(45) Veröffentlichungstag und Bekanntmachung des Hinweises auf die Patenterteilung: **30.09.2020**
(21) Anmeldenummer: 11749341.1
(22) Anmeldetag: 06.08.2011
(51) Int. Cl.: B01F 3/04, C12M 1/04

(54) **BEGASUNGSVORRICHTUNG FÜR BIOREAKTOREN**
GASSING DEVICE FOR BIOREACTORS
DISPOSITIF D'ALIMENTATION EN GAZ POUR DES BIORÉACTEURS

(30) Priorität: 30.09.2010 DE 102010046989
(43) Veröffentlichungstag der Anmeldung: 07.08.2013
(73) Patentinhaber: Sartorius Stedim Biotech GmbH, 37079 Göttingen (DE)
(72) Erfinder: WEISSHAAR, Stefan, 37139 Adelebsen (DE)
(74) Vertreter: Vigand, Philippe
(86) Internationale Anmeldenummer: PCT/EP2011/003953
(87) Internationale Veröffentlichungsnummer: WO 2012/041416

(56) Entgegenhaltungen:
- EP-A2- 0 829 534
- WO-A1-2011/057718
- US-A- 4 207 275
- US-A1- 2006 033 222

## Beschreibung

### Gebiet der Erfindung

Die Erfindung betrifft eine Begasungsvorrichtung für Bioreaktoren mit einem Begasungselement mit Gasaustrittsöffnungen.

### Stand der Technik

Die Gasversorgung und insbesondere die Sauerstoffversorgung ist ein wichtiger Schlüssel bei zellulären Stoffwechselvorgängen. Obwohl Tierzellkulturen wesentlich weniger Sauerstoff verbrauchen als Bakterien und Hefekulturen, ist die effiziente Versorgung die größte Herausforderung beim Betrieb eines Zellkultur-Bioreaktors. Neben der Sauerstoffversorgung der Zellen spielt auch die Konzentration von gelöstem Kohlendioxid als Regelgröße eine Rolle.

Es gibt zwei klassische Begasungsmethoden: Die Begasung des Kopfraumes des Bioreaktors und das direkte Einblasen der Gase durch Begasungsringe. Angewendet werden dabei sowohl die aus Fermentern bekannten Begasungsringe mit Bohrungen bzw. Gasaustrittsöffnungen von z.B. 0,8 mm als auch so genannte Mikrosparger aus gesinterten Kunststoffen mit Porengrößen von z.B. 20 bis 60 µm, die ebenfalls Gasaustrittsöffnungen bilden. Beide Typen haben spezielle Vor- bzw. Nachteile.

Der Begasungsring erzeugt größere Blasen, daher sind für die gleiche "Sauerstofftransferrate" größere Gasdurchsatzraten nötig. Der Ringsparger ist mit seinen relativ großen Blasen zum CO₂-Strippen bzw. Austreiben durch z.B. Luft geeignet.

Der Mikrosparger mit seinen relativ kleinen Blasen ist besonders zur Sauerstoffversorgung geeignet.

Nachteilig dabei ist jedoch, dass es wegen der relativ kleinen Blasen unter ungünstigen Bedingungen zur Schaumbildung kommen kann. Aufgrund seiner unterschiedlichen Porengrößen des gesinterten Materials, die zwangsläufig unmittelbar beabstandete Gasaustrittsöffnungen mit einer Größe zwischen etwa 20 bis etwa 70 µm bilden, erzeugt der Mikrosparger Gasblasen in einem relativ breiten Bereich von etwa 2 mm bis etwa 10 mm Durchmesser.

Aus der US 2006/033222 A1 ist eine Begasungsvorrichtung mit in einem Gehäuse angeordneten Begasungselementen bekannt, deren Gasaustrittsöffnungen in der Größe beliebig variieren. Mikrosparger, sowie deren konzentrische Anordnung innerhalb eines ringförmigen Scheibengehäuses, werden nicht offenbart.

Die Begasungsvorrichtungen werden als Teil von automatisierten Begasungssystemen von Bioreaktoren, beispielsweise Einwegreaktoren eingesetzt, wobei die Zufuhr von Luft, Sauerstoff, Kohlendioxid und Stickstoff unabhängig voneinander regelbar ist. Sensoren für den SauerstoffPartialdruck und den pH-Wert ermöglichen die Regelung dieser wichtigen Prozessparameter.

Aus der WO 2009/122310 A2, WO 2009/115926 A2 und der WO 2009/116002 A1 sind Einweg-Bioreaktoren mit einem Mischer und mit einer am Boden des Reaktorinnenraumes angeordneten Begasungsvorrichtung bekannt. Dabei ist es bekannt, am Boden zwei Begasungselemente anzuordnen, die als gegenüberliegende ineinander greifende Ringsegmente ausgebildet sind.

Soweit dabei Mikrosparger eingesetzt werden, weisen sie die oben beschriebenen Nachteile auf.

### Aufgabenstellung

Aufgabe der vorliegenden Erfindung ist es daher, die bekannten als Mikrosparger ausgebildeten Begasungsvorrichtungen für Bioreaktoren so zu verbessern, dass die Blasenbildung des als Mikrosparger ausgebildeten Begasungselementes bei einfachem Aufbau in einem enger und besser definierbaren Bereich ermöglicht wird.

### Darstellung der Erfindung

Diese Aufgabe wird in Verbindung mit dem Oberbegriff des Anspruches 1 dadurch gelöst, dass das Begasungselement als ein Mikrosparger ausgebildet ist, dessen Gasaustrittsöffnungen jeweils beabstandet zueinander angeordnet sind und eine Größe zwischen 100 µm und 200 µm aufweisen.

Während bei der bekannten Verwendung von porösem Material die mit einander unmittelbar beabstandeten Gasaustrittsöffnungen unterschiedliche Größen zwischen 20 bis 60 µm aufweisen, wird bei den beabstandeten Gasaustrittsöffnungen auf poröses Material verzichtet, so dass die Gasaustrittsöffnungen alle die gleiche vorgegebene Größe aufweisen, die zwischen 100 µm und 200 µm liegen kann.

Bevorzugt werden dabei für den Mikrosparger Gasaustrittsöffnungen mit einem Durchmesser zwischen 130 µm und 180 µm und nach einer weiteren bevorzugten Ausführungsform der Erfindung Gasaustrittsöffnungen mit einem Durchmesser von etwa 150 µm verwendet.

Überraschend hat sich für den Fachmann gezeigt, dass einander beabstandete Gasaustrittsöffnungen mit relativ großen Durchmessern von etwa 150 µm Gasblasen in einem relativ eng definierten Bereich von 2 bis 6 mm erzeugen können.

Das Gehäuse lässt sich durch den Verzicht auf poröses Material zudem erheblich vereinfachen.

Gemäß der Erfindung ist mindestens ein zweites Begasungselement mit Gasaustrittsöffnungen einer zweiten Größe vorgesehen, wobei die Begasungselemente von einem gemeinsamen Gehäuse mit getrennten Begasungskanälen gebildet werden.

Durch das gemeinsame Gehäuse lässt sich die Begasungsvorrichtung bei geringem Platzbedarf einfach und zentral zu einem Mischer des Bioreaktors anordnen.

Gemäß der Erfindung ist das Gehäuse als eine ringförmige Scheibe ausgebildet ist, in der die Begasungselemente konzentrisch angeordnet sind.

Die konzentrische Anordnung der Begasungselemente zueinander ermöglicht eine optimale Anordnung bezüglich eines Mischers bzw. Rührers, welcher wiederum die Gasblasen in einer derartigen Anordnung optimal verteilen kann.

Gemäß der Erfindung ist das zweite Begasungselement als ein Sparger oder Ringsparger ausgebildet, dessen Gasaustrittsöffnungen jeweils beabstandet zueinander angeordnet sind und eine Größe zwischen 600 µm und 1000 µm aufweisen. Bevorzugt weisen die Gasaustrittsöffnungen des als Sparger ausgebildeten zweiten Begasungselementes eine Größe von 800 µm auf. Dabei lassen sich Gasblasen mit einem Durchmesser zwischen 8 und 12 mm erzeugen.

Nach einer weiteren bevorzugten Ausführungsform der Erfindung weist das Gehäuse ein Unterteil auf, in dem die Begasungskanäle mit jeweils einem radial verlaufenden Zufluss angeordnet sind. Damit weist jeder der separat voneinander angeordneten Begasungskanäle einen eigenen radial verlaufenden Zufluss auf.

Gemäß einer weiteren bevorzugten Ausführungsform der Erfindung ist das Unterteil von einem Oberteil abgedeckt, das den Begasungskanal des Spargers und den Begasungskanal des Mikrospargers abdeckt und die Gasaustrittsöffnungen der Begasungselemente aufweist.

Das Oberteil lässt sich gegenüber dem Unterteil relativ einfach durch Dichtungen, beispielsweise Schnurringdichtungen bzw. O-Ringe, abdichten. Ebenso lässt sich das Oberteil mit dem Unterteil verkleben bzw. durch Verschweißen fest miteinander verbinden.

Gemäß einer weiteren bevorzugten Ausführungsform der Erfindung weist das Gehäuse eine zentrale auf einen Rührerflansch des Bioreaktors abgestimmte Öffnung auf und ist einem Rührer vorgelagert am Boden eines Innenraums des Bioreaktors anordenbar.

Durch die zentrale Öffnung der Begasungsvorrichtung ist diese optimal zu einem im Reaktorinnenraum angeordneten Rührer positionierbar.

Durch unterschiedliche Größen können die Volumenströme der Begasungselemente an das Reaktorvolumen angepasst werden. Insbesondere ist bei den Begasungselementen die Lochzahl auf das Reaktor- bzw. Nutzvolumen abstimmbar. Damit kann eine konstante Blasengrößenverteilung bei beliebiger Skalierbarkeit des Reaktorvolumens erreicht werden.

Weitere Einzelheiten der Erfindung ergeben sich aus der nachfolgenden ausführlichen Beschreibung und den beigefügten Zeichnungen, in denen bevorzugte Ausführungsformen der Erfindung beispielhaft veranschaulicht sind.

### Kurzbeschreibung der Zeichnungen

In den Zeichnungen zeigen:

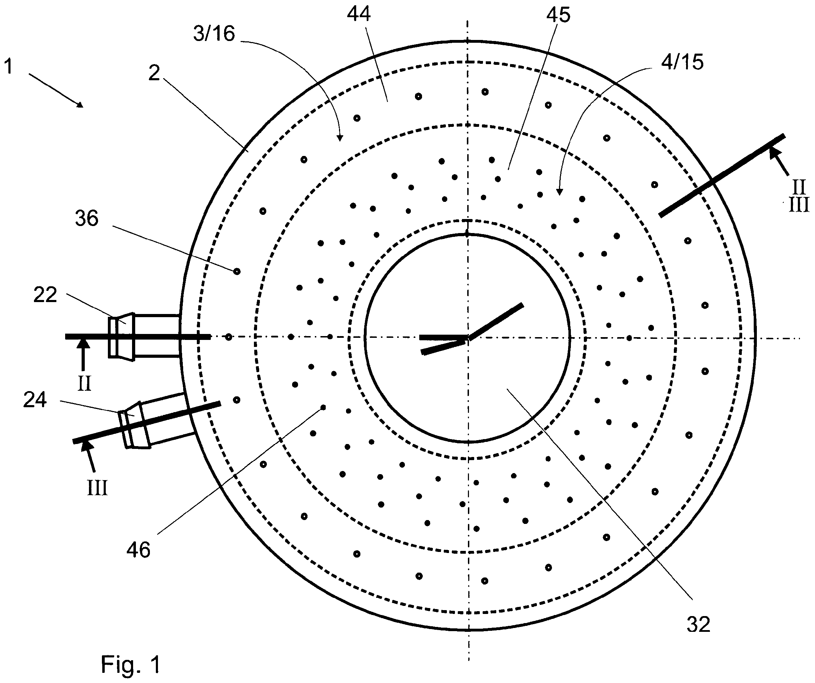

- Figur 1:: eine Draufsicht auf eine Begasungsvorrichtung mittlerer Größe in vergrößerter Darstellung,

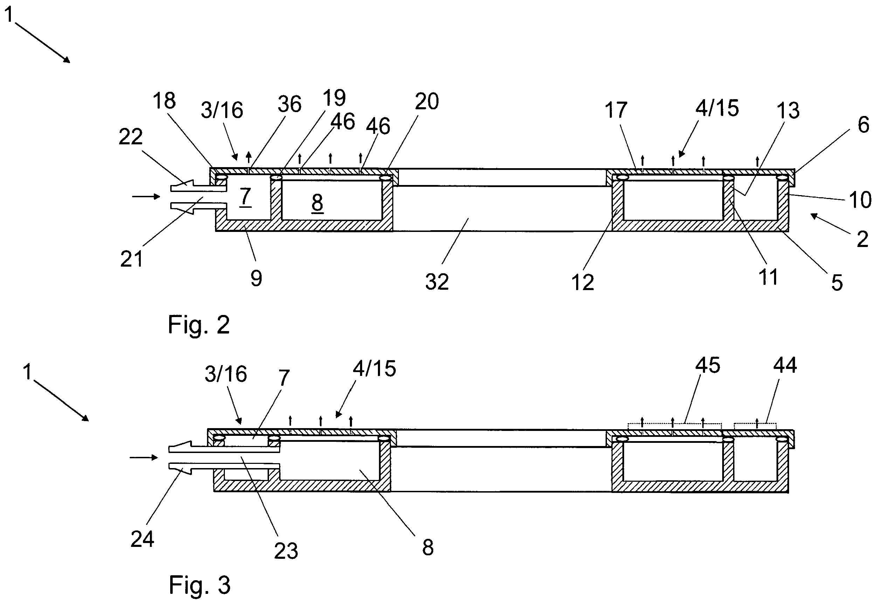

- Figur 2:: eine Seitenansicht der Begasungsvorrichtung von Fig. 1 entlang der Linie II - II geschnitten,
- Figur 3:: eine Seitenansicht der Begasungsvorrichtung von Fig. 1 entlang der Linie III - III geschnitten,
- Figur 4:: eine Seitenansicht in vergrößerter Darstellung einer Gasaustrittsöffnung mit einem Durchmesser von 800 µm eines als Sparger ausgebildeten Begasungselementes und austretenden Gasblasen,
- Figur 5:: ein Diagramm der Anzahl Gasblasen in Abhängigkeit von ihrer Größe (Durchmesser)nach Fig. 4,
- Figur 6:: eine Seitenansicht in vergrößerter Darstellung einer Gasaustrittsöffnung mit einem Durchmesser von 150 µm eines als Mikrosparger ausgebildeten Begasungselementes und austretenden Gasblasen,
- Figur 7:: ein Diagramm der Anzahl Gasblasen in Abhängigkeit von ihrer Größe (Durchmesser)nach Fig. 6,
- Figur 8:: eine Seitenansicht in vergrößerter Darstellung von Gasaustrittsöffnungen mit Durchmessern zwischen 20 und 60 µm eines als Mikrosparger mit porösem Material nach dem Stand der Technik ausgebildeten Begasungselementes und austretenden Gasblasen,
- Figur 9:: ein Diagramm der Anzahl Gasblasen in Abhängigkeit von ihrer Größe (Durchmesser) nach Fig. 8(Stand der Technik),
- Figur 10:: eine Draufsicht auf eine als Mikrosparger ausgebildeten Begasungsvorrichtung in vergrößerter Darstellung,
- Figur 11:: eine Seitenansicht der Begasungsvorrichtung von Fig. 10 entlang der Linie XI - XI geschnitten und
- Figur 12:: eine Seitenansicht eines Bioreaktors mit Rührer und einer Begasungsvorrichtung.

### Beschreibung der Ausführungsbeispiele

Eine Begasungsvorrichtung 1 besteht im Wesentlichen aus einem Gehäuse 2 mit einem (ersten) Begasungselement 4 und einem zweiten Begasungselement 3.

Das Gehäuse 2 besteht aus einem Unterteil 5 und einem auf das Unterteil 5 aufsetzbaren Oberteil 6. Das Unterteil 5 weist zwei konzentrisch zueinander angeordnete Begasungskanäle 7, 8 auf. Die Begasungskanäle 7, 8 sind in vertikaler Richtung nach unten von einem Boden 9 des Unterteils 5 begrenzt. Der zweite Begasungskanal 7 wird seitlich von der äußeren Wandung 10 und zum ersten Begasungskanal 8 hin von einer Zwischenwandung 11 begrenzt. Entsprechend wird der erste Begasungskanal 8 zum zweiten Begasungskanal 7 hin von der Zwischenwandung 11 und auf seiner dem zweiten Begasungskanal 7 abgewandten Seite durch eine innere Wandung 12 des Unterteils 5 begrenzt.

Dass auf das Unterteil 5 aufsetzbare Oberteil 6 verschließt mit einem zweiten ringförmigen Bereich 44 den zweiten Begasungskanal 7 und bildet mit diesem das zweite Begasungselement 3. Hierzu weist das Oberteil 6 in seinem zweiten ringförmigen Bereich 44 Gasaustrittsöffnungen 36 auf, deren Durchmesser jeweils etwa 800 µm beträgt. Das zweite Begasungselement 3 bildet dabei einen sogenannten Ringsparger bzw. Sparger 16.

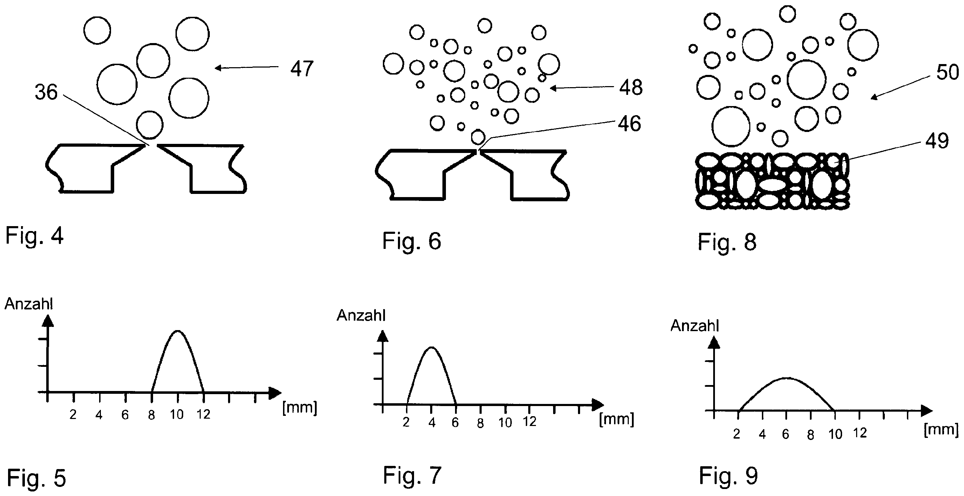

In Figur 4 ist eine Gasaustrittsöffnung 36 eines Spargers 16 mit austretenden Gasblasen 47 vergrößert dargestellt. Figur 5 zeigt die Verteilung unterschiedlicher Größen (8 bis 12 mm) der Gasblasen 47. Die Ordinate zeigt die Anzahl und die Abszisse die Größe.

Dass Oberteil 6 verschließt mit einem ersten ringförmigen Bereich 45 den ersten Begasungskanal 8 und bildet mit diesem das erste Begasungselement 4. Hierzu weist das Oberteil 6 in seinem ersten ringförmigen Bereich 45 Gasaustrittsöffnungen 46 auf, deren Durchmesser jeweils etwa 150 µm beträgt. Das erste Begasungselement 4 bildet dabei einen so genannten Mikrosparger 15.

In Figur 6 ist eine Gasaustrittsöffnung 46 eines Mikrospargers 15 mit austretenden Gasblasen 48 vergrößert dargestellt. Figur 7 zeigt die Verteilung unterschiedlicher Größen (2 bis 6 mm) der Gasblasen 48. Die Ordinate zeigt die Anzahl und die Abszisse die Größe.

In Figur 8 sind Gasaustrittsöffnungen eines aus dem Stand der Technik bekannten Mikrospargers mit porösem Material 49 mit austretenden Gasblasen 50 vergrößert dargestellt. Figur 9 zeigt die Verteilung unterschiedlicher Größen (2 bis 10 mm) der Gasblasen 50. Die Ordinate zeigt die Anzahl und die Abszisse die Größe.

Zur Abdichtung zwischen Unterteil 5 und Oberteil 6 sind auf den Wandungen 10, 11, 12 jeweils Dichtungen 18, 19, 20 angeordnet, die als Schnurringdichtungen bzw. O-Ringe ausgebildet sind.

Der zweite Begasungskanal 7 weist einen radial verlaufenden zweiten Zufluss 21 auf, der in einen Schlauchanschluss 22 mündet. Entsprechend weist der erste Begasungskanal 8 einen ersten radial verlaufenden Zufluss 23 auf, der in einen Schlauchanschluss 24 mündet. In den Schlauchanschlüssen 22, 24 oder in den Zuflüssen 21, 23 können nicht dargestellte Rückschlagventile angeordnet sein.

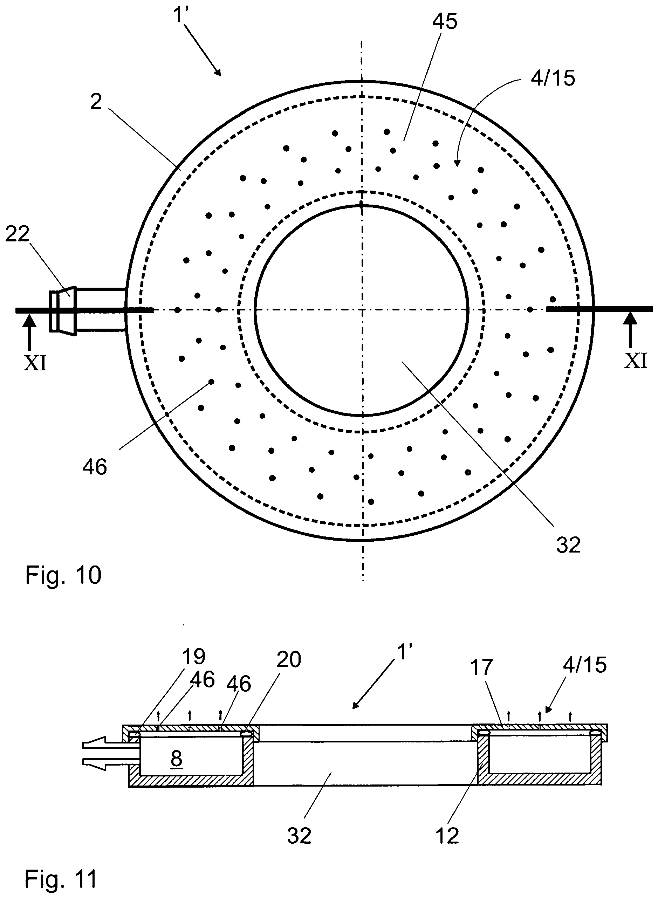

In den Figuren 10 und 11 ist eine als Mikrosparger 15 ausgebildete Begasungsvorrichtung 1' dargestellt.

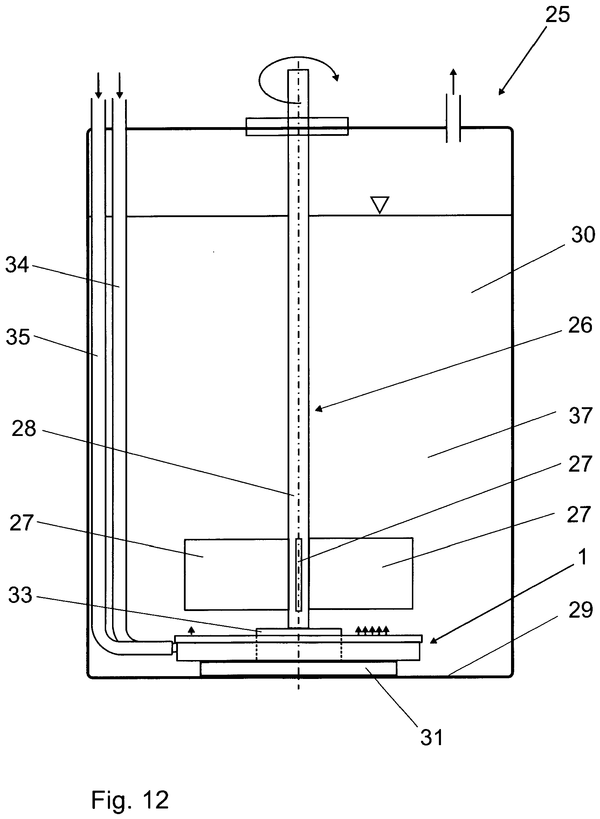

Figur 12 zeigt beispielhaft einen Biorektor 25, der beispielsweise als ein flexibler Beutel zum Einmalgebrauch ausgebildet ist und einen von außerhalb antreibbaren Rührer 26 mit Rührerblättern 27 aufweist. Der Bioreaktor 25 weist einen Innenraum 30 auf, an dessen Boden 29 ein Rührerflansch 31 angeordnet ist. In dem Rührerflansch 31 ist der Rührerschaft 28 drehbar gelagert. Mit ihrer zentralen Öffnung 32 ist die Begasungsvorrichtung 1 auf einen Bund 33 des Rührerflansches 31 aufgesteckt. Über eine zweite Zuflussleitung 34 wird dem zweiten Zufluss 21 und über eine erste Zuflussleitung 35 wird dem ersten Zufluss 23 Gas zugeführt, das aus der Begasungsvorrichtung 1 austritt und in dem flüssigen Medium 37 im Innenraum 30 Blasen bildet.

Weitere vorhandene Zu- und Abflüsse sowie Steuer- und Regeleinrichtungen sind nicht dargestellt.

## Patentansprüche

1. Begasungsvorrichtung (1, 1') für Bioreaktoren (25) mit einem in einem Gehäuse (2) angeordneten Begasungselement (4) mit Gasaustrittsöffnungen (46),
**dadurch gekennzeichnet,**
**dass** das Begasungselement (4) als ein Mikrosparger (15) ausgebildet ist, dessen Gasaustrittsöffnungen (46) jeweils beabstandet zueinander angeordnet sind und eine Größe zwischen 100 µm und 200 µm aufweisen,
**dass** mindestens ein zweites als Sparger (16) ausgebildetes Begasungselement (3, 7) mit Gasaustrittsöffnungen (36) einer zweiten Größe vorgesehen ist, dessen Gasaustrittsöffnungen (36) jeweils beabstandet zueinander angeordnet sind und eine Größe zwischen 600 µm und 1000 µm aufweisen,
**dass** die Begasungselemente (3, 4) von einem gemeinsamen Gehäuse (2) mit getrennten Begasungskanälen (7, 8) gebildet werden, und
**dass** das Gehäuse (2) als eine ringförmige Scheibe ausgebildet ist, in der die Begasungselemente (3, 4) konzentrisch angeordnet sind.

2. Begasungsvorrichtung nach Anspruch 1,
**dadurch gekennzeichnet,**
**dass** die Gasaustrittsöffnungen (46) des als Mikrosparger (15) ausgebildeten Begasungselementes (4) jeweils eine Größe zwischen 130 µm und 180 µm aufweisen.

3. Begasungsvorrichtung nach Anspruch 2,
**dadurch gekennzeichnet,**
**dass** die Gasaustrittsöffnungen (46) des als Mikrosparger (15) ausgebildeten Begasungselementes (4) jeweils eine Größe von 150 µm aufweisen.

4. Begasungsvorrichtung nach einem der vorhergehenden Ansprüche,
**dadurch gekennzeichnet,**
**dass** die Gasaustrittsöffnungen (36) des als Sparger (16) ausgebildeten zweiten Begasungselementes (7) eine Größe von 800 µm aufweisen.

5. Begasungsvorrichtung nach einem der Ansprüche 1 bis 4,
**dadurch gekennzeichnet,**
**dass** das Gehäuse (2) ein Unterteil (5) aufweist, in dem der oder die Begasungskanäle (7,8) mit jeweils einem radial verlaufenden Zufluss (21, 23) angeordnet sind.

6. Begasungsvorrichtung nach Anspruch 5,
**dadurch gekennzeichnet,**
**dass** das Unterteil (5) von einem Oberteil (6) abgedeckt ist, das den Begasungskanal (7) des Spargers (16) und den Begasungskanal (8) des Mikrospargers (15) abdeckt und die Gasaustrittsöffnungen (36, 46) der Begasungselemente (3, 4) aufweist.

7. Begasungsvorrichtung nach einem der Ansprüche 1 bis 6,
**dadurch gekennzeichnet,**
**dass** das Gehäuse (2) eine zentrale, auf einen Rührerflansch (31) eines Bioreaktors (25) abgestimmte Öffnung (32) aufweist, einem Rührer (26) vorgelagert und am Boden (29) eines Innenraums (30) des Bioreaktors (25) angeordnet ist.

## Claims

1. A gassing device (1, 1') for bioreactors (25) having a gassing element (4) arranged in a housing (2) with gas outlet openings (46),
**characterized**
**in that** the gassing element (4) is embodied as a microsparger (15) whose gas outlet openings (46) are each arranged spaced apart from one another and have a size between 100 µm and 200 µm,
**in that** at least a second gassing element (3, 7) embodied as a sparger (16) having gas outlet openings (36) of a second size is provided, whose gas outlet openings (36) are each arranged spaced apart from one another and have a size between 600 µm and 1000 µm,
**in that** the gassing elements (3, 4) are formed by a common housing (2) having separate gassing channels (7, 8), and
**in that** the housing (2) is embodied as an annular disk, in which the gassing elements (3, 4) are concentrically arranged.

2. The gassing device according to claim 1,
**characterized**
**in that** the gas outlet openings (46) of the gassing element (4) embodied as a microsparger (15) each have a size between 130 µm and 180 µm.

3. The gassing device according to claim 2,
**characterized**
**in that** the gas outlet openings (46) of the gassing element (4) embodied as a microsparger (15) each have a size of 150 µm.

4. The gassing device according to any of the preceding claims,
**characterized**
**in that** the gas outlet openings (36) of the second gassing element (7) embodied as a sparger (16) have a size of 800 µm.

5. The gassing device according to any of claims 1 to 4, **characterized**
**in that** the housing (2) has a lower part (5), in which the gassing channel or gassing channels (7, 8) are arranged in each case with a radially extending inflow (21, 23).

6. The gassing device according to claim 5,
**characterized**
**in that** the lower part (5) is covered by an upper part (6), which covers the gassing channel (7) of the sparger (16) and the gassing channel (8) of the microsparger (15) and the gas outlet openings (36, 46) of the gassing element (3, 4).

7. The gassing device according to any of claims 1 to 6, **characterized**
**in that** the housing (2) has a central opening (32) adapted to a stirrer flange (31) of a bioreactor (25), arranged upstream of a stirrer (26) and on the bottom (29) of an interior (30) of the bioreactor (25).

## Revendications

1. Dispositif de gazéification (1, 1') pour des bioréacteurs (25) avec un élément de gazéification (4) agencé dans un logement (2) et doté d'orifices de sortie de gaz (46),
**caractérisé**
**en ce que** l'élément de gazéification (4) est conçu en tant que microdiffuseur (15) dont les orifices de sortie de gaz (46) sont respectivement agencés à distance les uns des autres et présentent une dimension entre 100 µm et 200 µm,
**en ce qu'**au moins un second élément de gazéification (3, 7) conçu en tant que diffuseur (16) est prévu avec des orifices de sortie de gaz (36) d'une seconde dimension dont les orifices de sortie de gaz (36) sont respectivement agencés à distance les uns des autres et présentent une dimension entre 600 µm et 1 000 µm,
**en ce que** les éléments de gazéification (3, 4) sont formés d'un logement (2) commun avec des canaux de gazéification (7, 8) séparés, et
**en ce que** le logement (2) est conçu en tant que disque annulaire dans lequel les éléments de gazéification (3, 4) sont agencés de façon concentrique.

2. Dispositif de gazéification selon la revendication 1,
**caractérisé**
**en ce que** les orifices de sortie de gaz (46) de l'élément de gazéification (4) conçu en tant que microdiffuseur (15) présentent respectivement une dimension entre 130 µm et 180 µm.

3. Dispositif de gazéification selon la revendication 2,
**caractérisé**
**en ce que** les orifices de sortie de gaz (46) de l'élément de gazéification (4) conçu en tant que microdiffuseur (15) présentent respectivement une dimension de 150 µm.

4. Dispositif de gazéification selon l'une des revendications précédentes,
**caractérisé**
**en ce que** les orifices de sortie de gaz (36) du second élément de gazéification (7) conçu en tant que diffuseur (16) présentent une dimension de 800 µm.

5. Dispositif de gazéification selon l'une des revendications 1 à 4,
**caractérisé**
**en ce que** le logement (2) présente une partie inférieure (5) dans laquelle le ou les canaux de gazéification (7, 8) sont agencés avec respectivement un afflux (21, 23) qui s'étend radialement.

6. Dispositif de gazéification selon la revendication 5,
**caractérisé**
**en ce que** la partie inférieure (5) est recouverte par une partie supérieure (6) qui recouvre le canal de gazéification (7) du diffuseur (16) et le canal de gazéification (8) du microdiffuseur (15), et présente les orifices de sortie de gaz (36, 46) des éléments de gazéification (3, 4).

7. Dispositif de gazéification selon l'une des revendications 1 à 6,
**caractérisé**
**en ce que** le logement (2) présente un orifice (32) central ajusté à une flasque d'agitation (31) d'un bioréacteur (25), monté en amont d'un agitateur (26) et agencé au fond (29) d'un espace intérieur (30) du bioréacteur (25).
